(19) Europäisches Patentamt
European Patent Office
Office européen des brevets

(11) Veröffentlichungsnummer: **0 156 770**
**B1**

(12) **EUROPÄISCHE PATENTSCHRIFT**

(45) Veröffentlichungstag der Patentschrift: **12.10.88**

(21) Anmeldenummer: **85810121.5**

(22) Anmeldetag: **21.03.85**

(51) Int. Cl.⁴: **C 07 C 57/60**, C 07 C 69/65, C 07 C 121/70, C 07 C 147/06, C 07 C 51/353, C 07 C 67/347, C 07 C 120/00, C 07 C 57/42, C 07 C 69/618

(54) **Stilbenderivate.**

(30) Priorität: **27.03.84 CH 1526/84**

(43) Veröffentlichungstag der Anmeldung: **02.10.85 Patentblatt 85/40**

(45) Bekanntmachung des Hinweises auf die Patenterteilung: **12.10.88 Patentblatt 88/41**

(84) Benannte Vertragsstaaten: **CH DE FR GB LI**

(56) Entgegenhaltungen:
**CH-A-475 184**
**DE-A-2 531 456**
**DE-A-2 602 750**

(73) Patentinhaber: **CIBA- GEIGY AG, Klybeckstrasse 141, CH- 4002 Basel (CH)**

(72) Erfinder: **Artz, Klaus, Dr., Ahornstrasse 3, CH- 4132 Muttenz (CH)**
Erfinder: **Meyer, Hans Rudolf, Dr., Bollwerkstrasse 102, CH- 4102 Binningen (CH)**
Erfinder: **Reinehr, Dieter, Dr., Wolfsheule 10, CH- 7842 Kandern (CH)**
Erfinder: **Weber, Kurt, Dr., Rennweg 98, CH- 4052 Basel (CH)**

**EP 0 156 770 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die vorliegende Erfindung betrifft neue Stilbenderivate, ein Verfahren zu deren Herstellung sowie deren Verwendung als Zwischenprodukte zur Herstellung von optischen Aufhellern der Reihe der 4,4'-disubstituierten Stilbene.

Aus der DE-A-2 602 750 ist bekannt, 4,4'-Divinylstilbene aus substituierten Styrolen, Divinylbenzoinen, Stilbenen, welche in den 4 und 4'-Stellungen je eine aktivierte, gegebenenfalls substituierte Methylengruppe oder eine reaktionsfähige Carbonylgruppe oder ein funktionelles Derivat davon tragen, oder aus Fumarsäure-di-(p-vinylphenoxy)estern herzustellen. Die dabei als Ausgangsstoffe verwendeten Verbindungen sind schwer zugänglich (z. B. die Aldehyde) bzw. die gewählten Synthesen sehr umständlich. Aus der EP-A-0 040 581 ist bekannt 4,4'-Divinylstilbene aus Stilben-4,4'-dicarbonsäurechlorid und einem Olefin unter Zusatz eines Palladiumkatalysators herzustellen. Das als Ausgangsstoff verwendbare Stilben-4,4'-dicarbonsäurechlorid wird aus der schwer herstellbaren Stilben-4,4'-dicarbonsäure erhalten.

Es wurden nun neue Ausgangsstoffe zur Herstellung von optischen Aufhellern der Reihe der 4,4'-Divinylstilbene gefunden; die leicht zugänglich sind.

Die erfindungsgemässen Stilbenderivate entsprechen der Formel

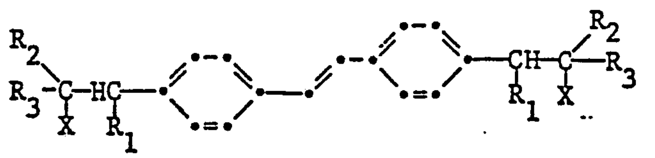

(1)

worin

X Chlor oder Brom,

$R_1$ Wasserstoff, Carboxyl, Cyano, $C_{1-6}$-Alkyl oder COOR, worin R fur $C_{1-4}$ Alkyl oder $C_{3-4}$-Alkenyl und

$R_2$ und $R_3$ unabhängig voneiander Wasserstoff, unsubstituiertes oder durch $C_{1-4}$-Alkoxy oder $C_{1-4}$-Alkoxycarbonyl substituiertes $C_{1-6}$-Alkyl oder $C_{3-4}$-Alkenyl oder Cyano, Carbonamid, $-COOR_o$

worin $R_o$ für Wasserstoff, $C_{1-4}$-Alkyl, Hydroxy-$C_{2-4}$-Alkyl, $C_{1-4}$-Alkoxy-$C_{2-4}$-Alkyl, $C_4$-Alkoxy-$C_{2-6}$-alkoxy-$C_{2-4}$-alkyl oder $C_{2-6}$-Hydroxyalkoxy-$C_{2-4}$-alkyl steht, oder $SO_2R_x$

worin $R_x$ für $C_{1-4}$-Alkyl, $C_{1-4}$-Alkoxy-$C_{2-4}$-alkyl, $C_{1-4}$-Alkoxy-$C_{2-4}$-alkoxy-$C_{2-4}$-alkyl, Phenyl oder Benzyl steht, bedeuten.

$C_{1-6}$-Alkylrestekönnen geradkettig oder verzweigt sein. Bevorzugt sind solche mit 1-4 und besonders 1 oder 2 C-Atomen. $C_{3-4}$-Alkenylgruppen weisen vorzugsweise 3 C-Atome auf. Als Beispiele derartiger Alkyl- und Alkenylgruppen seien erwähnt:

die Methyl-, Äthyl-, n-Propyl-, Isopropyl-, Butyl-, sec.-Butyl-, tert.-Butyl-, n-Pentyl-, n-Hexyl-, Allyl- und Butenylgruppe.

Als nicht chromophore Substituenten an definitionsgemässen Alkyloder Alkenylgruppen kommen z. B. Alkoxy-, Hydroxyalkoxy oder Alkoxycarbonylgruppen in Betracht. Alkoxyteile in den genannten Substituenten enthalten vorzugsweise 1 bis 6 und besonders 1 oder 2 C-Atome.

Nicht chromophore Substituenten 2. Ordnung sind elektronenanziehende Substituenten, die den Verbindungen der Formel (I) keine Farbe werleihen, was deren Verwendung zur Herstellung von optischen Aufhellern beeinträchtigen würde. Als Beispiele seien genannt: die Cyanogruppe, die Carbonamidgruppe, die Carboxylgruppe, Carbonsäureestergruppen und Sulfonylgruppen. Nicht chromophor veresterte Carboxylgruppen sind z. B. Carbonsäurealkyl- oder Carbonsäurealkenylestergruppen, wobei das Alkyl bzw. das Alkenyl wie oben erwähnt substituiert sein kann. Carbonsäureestergruppen sind vorzugsweise Carbonsäurealkylestergruppen, wobei das Alkyl durch Hydroxy oder Alkoxy-, Alkoxyalkoxy- oder Hydroxyalkoxygruppen der oben erwähnten Art substituiert sein kann. Sulfonylgruppen sind vorzugsweise Alkyl-, Phenyl- oder Benzylsulfonylgruppen, wobei das Alkyl durch Alkoxy- oder Alkoxyalkoxygruppen der oben erwähnten Art substituiert sein kann.

Bevorzugt sind Stilbenderivate der Formel (I) worin

$R_1$ Wasserstoff, $C_{1-4}$-Alkyl oder COOR, worin R für $C_{1-4}$-Alkyl oder $C_{3-4}$-Alkenyl steht,

$R_2$ Wasserstoff, $C_{1-4}$-Alkyl, durch $C_{1-4}$-Alkoxy oder $C_{1-4}$-Alkoxycarbonyl substituiertes $C_{1-4}$-Alkyl, $C_{3-4}$-Alkenyl, Cyano, $-COOR_o$

worin $R_o$ für Wasserstoff, $C_{1-4}$-Alkyl, Hydroxy-$C_{2-4}$-Alkyl, $C_{1-4}$-Alkoxy-$C_{2-4}$-Alkyl, $C_{1-4}$-Alkoxy-$C_{2-6}$-alkoxy-$C_{2-4}$-alkyl oder $C_{2-6}$-Hydroxyalkoxy-$C_{2-4}$-alkyl steht, oder $SO_2R_x$

worin $R_x$ für $C_{1-4}$-Alkyl, $C_{1-4}$-Alkoxy-$C_{2-4}$-alkyl, $C_{1-4}$-Alkoxy-$C_{2-4}$-alkoxy-$C_{2-4}$-alkyl, Phenyl oder Benzyl steht, und

$R_3$ Wasserstoff, $C_{1-4}$-Alkyl oder durch $C_{1-4}$-Alkoxy oder $C_{1-4}$-Alkoxycarbonyl substituiertes $C_{1-4}$-Alkyl, bedeuten.

Des weiteren bevorzugt sind Stilbenderivate der Formel (I), worin

X Chlor,

$R_1$ Wasserstoff oder $C_{1-2}$-Alkyl,

$R_2$ $C_{3-4}$-Alkenyl, Cyano oder $-COOR_o$ oder $-SO_2R_x$ worin $R_o$ für Wasserstoff oder $C_{1-4}$-Alkyl und $R_x$ für $C_{1-4}$-Alkyl stehen und

$R_3$ Wasserstoff oder $C_{1-4}$-Alkyl, bedeuten.

Besonders bevorzugt sind Stilbenderivate der Formel (I) worin

$R_1$ Wasserstoff,

$R_2$ Cyano oder $-COOR_o$ oder $-SO_2R_x$ worin $R_o$ für Wasserstoff, Methyl oder Äthyl und $R_x$ für Methyl oder Äthyl stehen und

$R_3$ Wasserstoff, bedeuten,

sowe das Stilbenderivat der Formel (I), worin $R_1$ und $R_3$ Wasserstoff und $R_2$ Cyano bedeuten.

Die Stilbenderivate der Formel (I) können in an sich bekannter Weise hergestellt werden, indem man 1 Mol einer Bis-diazoniumverbindung der Formel

(2) 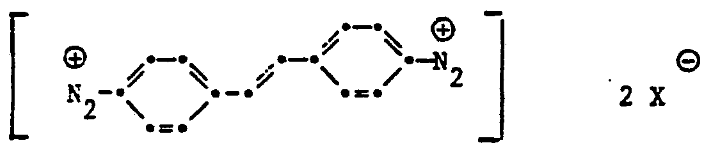

worin X Chlor oder Brom bedeutet, mit 2-8 und vorzugsweise 3-6 Mol einer Verbindung der Formel

(3) 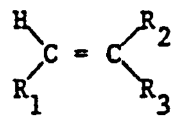

worin $R_1$, $R_2$ und $R_3$ die weiter oben angegebene Bedeutung haben, in Anwesenheit eines Kupfer enthaltenden Stoffes als Katalysator wie z. B. Kupferpulver, Kupferoxid, Kupfersalze oder deren Mischungen und in einem mit Wasser mischbaren Lösungsmittel umsetzt.

Als Katalysator verwendet man vorzugsweise ein Kupfer(I)-halogenid und besonders das Kupfer(I)-chlorid.

Die Reaktion wird bei einem pH von 1-5, vorzugsweise 1-2 vorgenommen.

Als mit Wasser mischbare Lösungsmittel kommen z. B. Aceton, Methyläthylketon, Acetonitril, N-Methylpyrrolidon, $C_{1-4}$-Alkanole, Dimethylformamid und Essigsäure in Betracht. Die Reaktion kann auch in Wasser, ohne weitere Lösungsmittelzusätze durchgeführt werden.

Die Temperatur, bei welcher die Umsetzung durchgeführt wird, liegt zwischen -10 und +50° C. Der Temperatur-Vorzugsbereich liegt bei 0 bis 30° C.

Verbindungen der Formel (I) worin $R_2$ Cyano oder $-COOR_o$ bedeuten, können auch hergestellt werden durch Austausch eines dieser Substituenten durch einen andern. So erhält man Carbonsäureester aus den entsprechenden Nitrilen nach bekannten Methoden über die Iminoäther (vgl. Houben-Weyl Bd.8, Seite 536) oder durch säurekatalysierte Veresterung bzw. Umesterung (vgl. Houben-Weyl, Bd.8, Seite 516, 528). Zu den Carbonsäuren gelangt man durch saure Verseifung der Nitrile oder Carbonsäureester (vgl. Houben-Weyl, Bd.8, Seite 418).

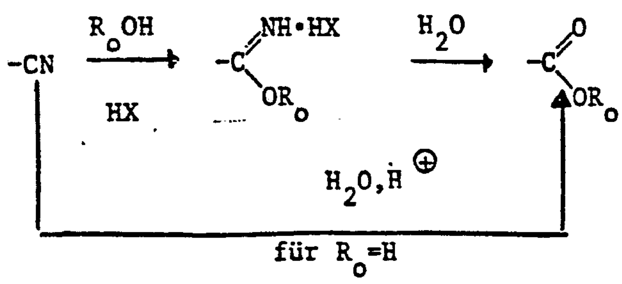


Die Stilbenverbindungen der Formel (I) können zur Herstellung von bekannten optischen Aufhellern der Reihe der 4,4'-disubstituierten Stilbene wie z. B. solche der US-A-4 108 887 verwendet werden. Durch Behandlung mit Basen erhält man unter Halogenwasserstoffabspaltung die, α, β-ungesättigten Verbindungen der Formel

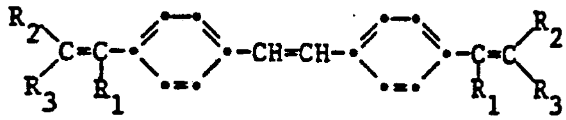

worin $R_1$, $R_2$ und $R_3$ die weiter oben angegebene Bedeutung haben.

Als Basen verwendet man beispielsweise Alkali- oder Erdalkalimetall-hydroxide oder -carbonate, vorzugsweise tertiäre Amine wie Trialkylamine oder Pyridine. Durch den Einsatz von tert. Aminen in wasserfreiem Medium kann eine mögliche Umwandlung der Ester-oder Nitrilgruppen vermieden werden. Verwendet nan dagegen Basen wie Alkalimetall-hydroxide, Ammoniak, primäre oder sekundäre Amine zur Halogenwasserstoffabspaltung, so erhält man die, α, β-ungesättigten Dicarbonsäuren bzw. deren Amide.

In den nachfolgenden Beispielen, welche die Erfindung näher erläutern, ohne sie darauf zu beschränken, sind Teile- und Prozentangaben immer, soweit nichts anderes angegeben ist, Gewichtsteile und Gewichtsprozente. Schmelz- und Siedepunkte sind, sofern nichts anderes angegeben ist, unkorrigiert.

**Beispiel 1**

21 g 4,4'-Diaminostilben werden in einer Mischung von 56 m Eisessig, 50 ml konz. Salzsäure und 6 ml Wasser gelöst. Das Reaktionsgemisch wird auf 0° C abgekühlt und innerhalb von 20 Minuten mit einer Lösung von 15,2 g Natriumnitrit in 30 ml Wasser versetzt. Die Temperatur soll dabei nicht über 5° C ansteigen. Die Lösung wird dann über eine Glasfritte filtriert.

31,9 g Acrylnitril werden in 24 ml Methyläthylketon vorgelegt und auf 10° C gekühlt. Innerhalb eines Zeitraumes von einer Stunde werden gleichzeitig die Lösung des Diazoniumsalzes und eine Lösung von 1,5 g Kupfer(I)-chlorid in 15 ml konz. Salzsäure zugetropft. Die Temperatur in der Reaktionsmischung wird durch externe Kühlung auf 10 - 20° C gehalten. Nach Beendigung der Stickstoffentwicklung wird der Niederschlag abfiltriert und mit Wasser neutral gewaschen.

Das braune kristalline Rohprodukt (30,6 g; Fp. 120 - 121° C) wird in ca. 1 Liter Ligroin mehrmals aufgekocht und die Ligroimphase abdekantiert, wobei das gelbliche weisse, kristalline Produkt aus der Ligroinlösung auskristallisiert. Es werden 18,3 g der Verbindung der Formel

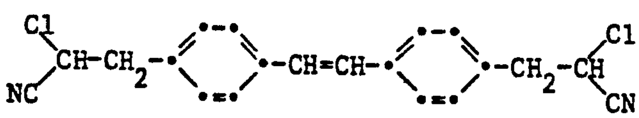

(100)

als kristallines Produkt von Smp. 128 - 132° C erhalten.

Verwendet man anstelle von Acrylnitril die entsprechende Menge Methylvinyl-sulfon und verzichtet auf die Beigabe von Methyläthylketon als Lösungsmittel, so erhält man die Verbindung der Formel

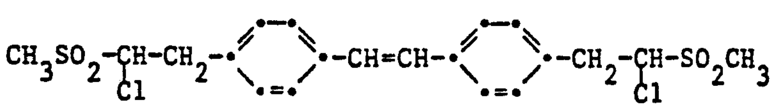

(101)

Smp. 268 - 272° C (nach Umkristallisation aus Äthylenglykolmonomethyläther).

In ähnlicher Weise erhält man aus Acrylsäureäthylester bzw. Acrylsäure die Verbindungen der Formel (102) und (103).

**Beispiel 2**

Zu einer Lösung von 3,55 g des Rohproduktes der Formel (100) in 40 ml Methylenchlorid fügt man 15 ml wasserfreies Äthanol umd leitet unter Rühren bei 0° C Salzsäuregas bis zur Sättigung ein. Nach Stehenlassen während 16 Stunden bei Raumtemperatur wird im Vakuum bei Raumtemperatur zur Trockene eingedampft und der Rückstand in 40 ml Methylenchlorid verrührt. Man nutschtdas unlösliche Iminoäther-hydrochlorid ab, wäscht es mit Methylenchlorid und trocknet es kurz im Vakuum bei Raumtemperatur (2,1 g). Dieses wird in einer Mischung von 10 ml Wasser und 10 ml Äthanol während 30 Minuten bei Raumtemperatur verrührt. Das Reaktionsprodukt wird genutscht, wiederholt mit Wasser gewaschen und im Vakuum bei 50° C getrocknet. Man erhält 1,3 g der Verbindung der Formel

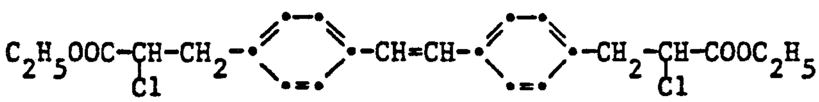 (102)

Smp. 95 - 97°C nach Umkristallisation aus Isopropanol und Cyclohexan.

**Beispiel 3**

1,8 g des Rohproduktes der Formel (100) werden in 12 ml Ameisensäure und 8 ml konz. Salzsäure 2 Stunden unter Rückfluss erhitzt. Nachdem das Ausgangsprodukt in Lösung gegangen ist, fällt das Reaktionsprodukt aus. Dieses wird bei Raumtemperatur abgenutscht, wiederholt mit Wasser gewaschen und im Vakuum bei 100°C getrocknet. Man erhält 1,65 g der Verbindung der Formel

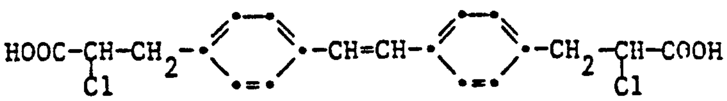 (103)

Smp. 204 - 211°C nach Umkristallisieren aus Chlorbenzol.

**Beispiel 4**

6 g der Verbindung der Formel (100) werden unter Rühren in 50 ml Dimethylformamid bei Raumtempetatur gelöst. Nach Zugabe von 6,2 ml Triäthylamin wird auf 75°C erwärmt und während 5 stunden bei dieser Temperatur gerührt. Zur entstandenen rohen Suspension werden 200 ml Methanol gegeben, abkühlen gelassen, das auskristallisierte Produkt abgenutscht, mit Methanol gewaschen und unter Vakuum getrocknet. Man erhält so 3,3 g des optischen Aufhellers der Formel

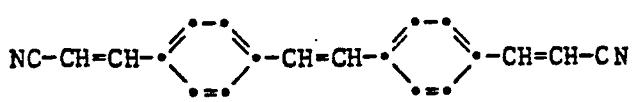 (200)

vom Schmelzpunkt: 192 - 195°C.

In analoger Weise erhält man aus dem Diäthylester der Formel (102) den optischen Aufheller der Formel

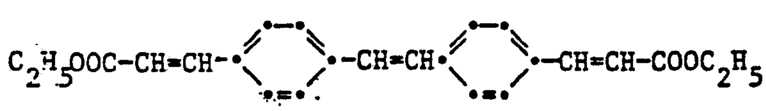 (201)

vom Smp. 200°C.

Erhitzt nan den Diäthylester der Formel (102) oder die Dicarbonsäure der Formel (103) mit überschüssiger, wässriger, alkoholischer Natronlauge bei Rückflusstemperatur, so erhält man nach dem Ansäuern die Verbindung der Formel

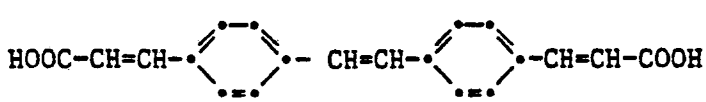 (202)

vom Smp. > 300°C.

**Patentansprüche**

1. Stilbenderivate der Formel

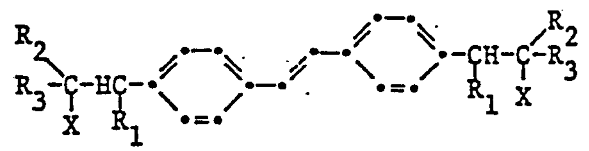

worin
X Chlor oder Brom,
$R_1$ Wasserstoff, Carboxyl, Cyano, $C_{1-6}$-Alkyl oder COOR, worin R für $C_{1-4}$-Alkyl oder $C_{3-4}$-Alkenyl und
$R_2$ und $R_3$ unabhängig voneinander Wasserstoff, unsubstituiertes oder durch $C_{1-4}$-Alkoxy oder $C_{1-4}$-Alkoxycarbonyl substituiertes $C_{1-6}$-Alkyl oder $C_{3-4}$-Alkenyl oder Cyano, Carbonamid, $-COOR_o$
worin $R_o$ für Wasserstoff, $C_{1-4}$-Alkyl, Hydroxy-$C_{2-4}$-Alkyl, $C_{1-4}$-Alkoxy-$C_{2-4}$-Alkyl, $C_{1-4}$-Alkoxy-$C_{2-6}$-alkoxy-$C_{2-4}$-alkyl oder $C_{2-6}$-Hydroxyalkoxy-$C_{2-4}$-alkyl steht, oder $SO_2R_x$
worin $R_x$ für $C_{1-4}$-Alkyl, $C_{1-4}$-Alkoxy-$C_{2-4}$-alkyl, $C_{1-4}$-Alkoxy-$C_{2-4}$-alkoxy-$C_{2-4}$-alkyl, Phenyl oder Benzyl steht, bedeuten.

2. Stilbenderivate gemäss Anspruch 1, worin
$R_1$ Wasserstoff, $C_{1-4}$-Alkyl oder COOR, worin R für $C_{1-4}$-Alkyl oder $C_{3-4}$-Alkenyl steht,
$R_2$ Wasserstoff, $C_{1-4}$-Alkyl, durch $C_{1-4}$-Alkoxy oder $C_{1-4}$-Alkoxycarbonyl substituiertes $C_{1-4}$-Alkyl, $C_{3-4}$-Alkenyl, Cyano, $-COOR_o$
worin $R_o$ für Wasserstoff, $C_{1-4}$-Alkyl, Hydroxy-$C_{2-4}$-Alkyl, $C_{1-4}$-Alkoxy-$C_{2-4}$-Alkyl, $C_{1-4}$-Alkoxy-$C_{2-6}$-alkoxy-$C_{2-4}$-alkyl oder $C_{2-6}$-Hydroxyalkoxy-$C_{2-4}$-alkyl steht, oder $SO_2R_x$
worin $R_x$ für $C_{1-4}$-Alkyl, $C_{1-4}$-Alkoxy-$C_{2-4}$-alkyl, $C_{1-4}$-Alkoxy-$C_{2-4}$-alkoxy-$C_{2-4}$-alkyl, Phenyl oder Benzyl steht, und
$R_3$ Wasserstoff, $C_{1-4}$-Alkyl oder durch $C_{1-4}$-Alkoxy oder $C_{1-4}$-Alkoxycarbonyl substituiertes $C_{1-4}$-Alkyl, bedeuten.

3. Stilbenderivate gemäss Anspruch 2, worin
X Chlor,
$R_1$ Wasserstoff oder $C_{1-2}$-Alkyl,
$R_2$ $C_{3-4}$-Alkenyl, Cyano oder $-COOR_o$ oder $-SO_2R_x$ worin $R_o$ für Wasserstoff oder $C_{1-4}$-Alkyl und $R_x$ für $C_{1-4}$-Alkyl stehen und
$R_3$ Wasserstoff oder $C_{1-4}$-Alkyl, bedeuten.

4. Stilbenderivate gemäss Anspruch 3, worin
$R_1$ Wasserstoff,
$R_2$ Cyano oder $-COOR_o$ oder $-SO_2R_x$, worin $R_o$ Wasserstoff, Methyl oder Äthyl und $R_x$ für Methyl oder Äthyl stehen und
$R_3$ Wasserstoff, bedeuten.

5. Stilbenderivat gemäss Anspruch 4, worin $R_1$ und $R_3$ Wasserstoff und $R_2$ Cyano bedeuten.

6. Verfahren zur Herstellung von Stilbenderivaten der Formel

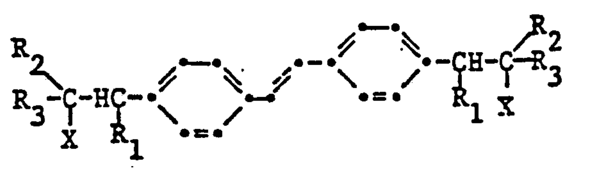

worin
X Chlor oder Brom,
$R_1$ Wasserstoff, Carboxyl, Cyano $C_{1-6}$-Alkyl oder COOR, worin R für $C_{1-4}$-Alkyl oder $C_{3-4}$-Alkenyl und
$R^2$ und $R_3$ unabhängig voneinander Wasserstoff, unsubstituertes oder durch
$C_{1-4}$-Alkoxy oder $C_{1-4}$-Alkoxycarbonyl substituiertes $C_{1-6}$-Alkyl oder
$C_{3-4}$-Alkenyl oder Cyano, Carbonamid, $-COOR_o$ worin $R_o$ für Wasserstoff,
$C_{1-4}$-Alkyl, Hydroxy-$C_{2-4}$-Alkyl $C_{1-4}$-Alkoxy-$C_{2-4}$-Alkyl $C_{1-4}$-Alkoxy-$C_{2-6}$-alkoxy-$C_{2-4}$-alkyl oder $C_{2-6}$-Hydroxyalkoxy-$C_{2-4}$-alkyl steht, oder $SO_2R_x$ worin $R_x$ für $C_{1-4}$-Alkyl, $C_{1-4}$-Alkoxy-$C_{2-4}$-alkyl, $C_{1-4}$-Alkoxy-$C_{2-4}$-alkoxy-$C_{2-4}$-alkyl, Phenyl oder Benzyl steht, bedeuten,
dadurch gekennzeichnet, dass man 1 Mol einer Bis-diazoniunverbindung der Formel

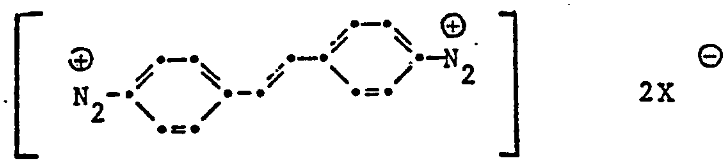

worin X Chlor oder Brom bedeutet, mit 2 - 8 und vorzugsweise 3 - 6 Mol einer Verbindung der Formel

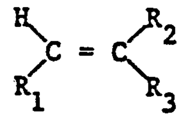

worin $R_1$, $R_2$ und $R_3$ die weiter oben angegebene Bedeutung haben, in Anwesenheit eines Kupfer-halogenids als Katalysators und in einem mit Wasser mischbaren Lösungsmittel umsetzt.

7. Verfahren gemäss Anspruch 6, worin als Katalysator Kupfer (I)-chlorid verwendet wird.

8. Verwendung der gemäss Anspruch 6 erhältlichen Stilbenderivate als Ausgangsprodukte für die Herstellung von optischen Aufhellern der Reihe der 4,4'-disubstituierten Stilbene.

## Claims

1. A stilbene derivative of the formula

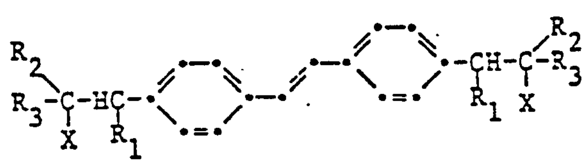

wherein

X is chlorine or bromine

$R_1$ is hydrogen, carboxyl, cyano, $C_1$-$C_6$alkyl or COOR, in which R is $C_1$-$C_4$alkyl or $C_3$-$C_4$alkenyl and

$R_2$ and $R_3$ independently of one another are each hydrogen, $C_1$-$C_6$alkyl which is unsubstituted or substituted by $C_1$-$C_4$alkoxy or $C_1$-$C_4$alkoxycarbonyl, or is $C_3$-$C_4$alkenyl or cyano, carboxamide, -$COOR_o$,

in which $R_o$ is hydrogen $C_1$-$C_4$alkyl, hydroxy-$C_2$-$C_4$alkyl $C_1$-$C_4$alkoxy-$C_2$-$C_4$alkyl, $C_1$-$C_4$alkoxy-$C_2$-$C_6$alkoxy-$C_2$-$C_4$alkyl or $C_2$-$C_6$-hydroxyalkoxy-$C_2$-$C_4$alkyl, or $SO_2R_x$,

in which $R_x$ is $C_1$-$C_4$alkyl, $C_1$-$C_4$alkoxy-$C_2$-$C_4$alkyl, $C_1$-$C_4$alkoxy-$C_2$-$C_4$alkoxy-$C_2$-$C_4$alkyl, phenyl or benzyl.

2. A stilbene derivative according to claim 1, wherein

$R_1$ is hydrogen, $C_1$-$C_4$alkyl or COOR, in which R is $C_1$-$C_4$alkyl or $C_3$-$C_4$alkenyl,

$R_2$ is hydrogen, $C_1$-$C_4$alkyl, $C_1$-$C_4$alkyl substituted by $C_1$-$C_4$alkoxy or $C_1$-$C_4$alkoxycarbonyl, or is $C_3$-$C_4$alkenyl, cyano, -$COOR_o$

in which $R_o$ is hydrogen, $C_1$-$C_4$alkyl hydroxy-$C_2$-$C_4$alkyl, $C_1$-$C_4$alkoxy-$C_2$-$C_4$alkyl, $C_1$-$C_4$alkoxy-$C_2$-$C_6$alkoxy-$C_2$-$C_4$alkyl or $C_2$-$C_6$hydroxyalkoxy-$C_2$-$C_4$alkyl, or $SO_2R_x$,

in which $R_x$ is $C_1$-$C_4$alkyl $C_1$-$C_4$alkoxy-$C_2$-$C_4$alkyl- $C_1$-$C_4$-alkoxy-$C_2$-$C_4$alkoxy-$C_2$-$C_4$alkyl, phenyl or benzyl, and

$R_3$ is hydrogen, $C_1$-$C_4$alkyl, or $C_1$-$C_4$alkyl substituted by $C_1$-$C_4$-alkoxy or $C_1$-$C_4$alkoxycarbonyl.

3. A stilbene derivative according to claim 2 wherein

X is chlorine,

$R_1$ is hydrogen or $C_1$-$C_2$alkyl,

$R_2$ is $C_3$-$C_4$alkenyl, cyano or -$COOR_o$ or -$SO_2R_x$, in which $R_o$ is hydrogen or $C_1$-$C_4$alkyl and $R_x$ is $C_1$-$C_4$alkyl, and

$R_3$ is hydrogen or $C_1$-$C_4$alkyl.

4. A stilbene derivative according to claim 3, wherein

$R_1$ is hydrogen,

$R_2$ is cyano or -$COOR_o$ or -$SO_2R_x$, in which $R_o$ is hydrogen, methyl or ethyl, and $R_x$ is methyl or ethyl, and

$R_3$ is hydrogen.

5. A stilbene derivative according to claim 4, wherein $R_1$ and $R_3$ are hydrogen and $R_2$ is cyano.

6. A process for producing a stilbene derivative of the formula

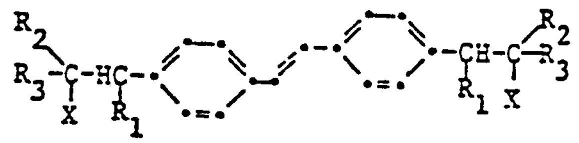

wherein

X is chlorine or bromine

$R_1$ is hydrogen, carboxyl, cyano, $C_1$-$C_6$alkyl or COOR, in which R is $C_1$-$C_4$alkyl or $C_3$-$C_4$alkenyl and

$R_2$ and $R_3$ independently of one another are each hydrogen, $C_1$-$C_6$alkyl which is unsubstituted or substituted by $C_1$-$C_4$alkoxy or $C_1$-$C_4$alkoxycarbonyl, or is $C_3$-$C_4$alkenyl or cyano, carboxamide, -$COOR_o$,

in which $R_o$ is hydrogen, $C_1$-$C_4$alkyl, hydroxy-$C_2$-$C_4$alkyl, $C_1$-$C_4$-alkoxy-$C_2$-$C_4$alkyl, $C_1$-$C_4$alkoxy-$C_2$-$C_6$alkoxy-$C_2$-$C_4$-alkyl or $C_2$-$C_6$-hydroxyalkoxy-$C_2$-$C_4$alkyl, or $SO_2R_x$,

in which $R_x$ is $C_1$-$C_4$alkyl, $C_1$-$C_4$alkoxy-$C_2$-$C_4$alkyl, or $C_1$-$C_4$alkoxy-$C_2$-$C_4$alkoxy-$C_2$-$C_4$-alkyl, phenyl or benzyl, which process comprises reacting 1 mol of a bis-diazonium compound of the formula

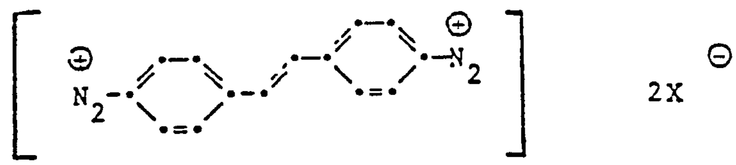

wherein X is chlorine or bromine, with 2 - 8, preferably 3 - 6, mol of a compound of the formula

wherein $R_1$ $R_2$ and $R_3$ have the meanings defined above, in the presence of a copper halide as a catalyst, and in a solvent miscible with water.

7. A process according to claim 6 wherein the catalyst used is copper (I) chloride.

8. Use of a stilbene derivative obtainable according to claim 6 as a starting product for producing optical brighteners of the 4,4'-disubstituted stilbene series.

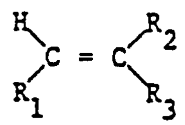

**Revendications**

1. Dérivés de stilbène de formule:

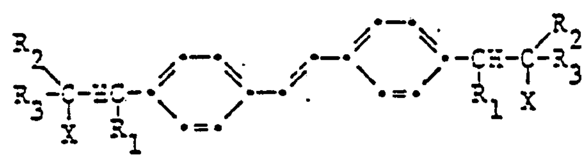

dans laquelle:

X représente un atome de chlore ou de brome,

$R_1$ représente un atome d'hydrogène ou un groupe carboxyle, cyano, alkyle en $C_{1-6}$, ou COOR, R représentant un groupe alkyle en $C_{1-4}$ ou alcényle en $C_{3-4}$, et

$R_2$ et $R_3$, indépendamment l'un de l'autre, représentent chacun un atome d'hydrogène ou un groupe alkyle en $C_{1-6}$ ou alcényle en $C_{3-4}$, non substitué ou substitué par un groupe alcoxy en $C_{1-4}$ ou (alcoxy en $C_{1-4}$)-carbonyle, ou bien un groupe cyano, carbonamido, -$COOR_o$,

$R_o$ représentant un atome d'hydrogène ou un groupe alkyle en $C_{1-4}$, hydroxyalkyle en $C_{2-4}$, (alcoxy en $C_{1-4}$)-alkyle en $C_{2-4}$, (alcoxy en $C_{1-4}$)-(alcoxy en $C_{2-6}$)-alkyle en $C_{2-4}$ ou (hydroxyalcoxy en $C_{2-6}$)-alkyle en $C_{2-4}$, ou bien encore un groupe $SO_2R_x$,

$R_x$ représentant un groupe alkyle en $C_{1-4}$, (alcoxy en $C_{1-4}$)-alkyle en $C_{2-4}$,(alcoxy en $C_{1-4}$)-(alcoxy en $C_{2-4}$)-alkyle en $C_{2-4}$, phényle ou benzyle.

2. Dérivés de stilbène conformes à la revendication 1, dans lesquels:

$R_1$ représente un atome d'hydrogène, un groupe alkyle en $C_{1-4}$, ou COOR, dans lequel R représente un groupe alkyle en $C_{1-4}$ ou alcényle en $C_{3-4}$,

$R_2$ représente un atome d'hydrogène ou un groupe alkyle en $C_{1-4}$, alkyle en $C_{1-4}$ substitué par un groupe alcoxy en $C_{1-4}$ ou (alcoxy en $C_{1-4}$)-carbonyle, alcényle en $C_{3-4}$, cyano, -$COOR_o$

dans lequel $R_o$, représente un atome d'hydrogène ou un groupe alkyle en $C_{1-4}$, hydroxyalkyle en $C_{1-4}$, (alcoxy en $C_{1-4}$)-alkyle en $C_{2-4}$, (alcoxy en $C_{1-4}$)-(alcoxy en $C_{2-6}$)-alkyle en $C_{2-4}$ ou (hydroxyalcoxy en $C_{2-6}$)-alkyle en $C_{2-4}$, ou encore $SO_2R_x$

dans lequel $R_x$ représente un groupe alkyle en $C_{1-4}$, (alcoxy en $C_{1-4}$)-alkyle en $C_{2-4}$, (alcoxy en $C_{1-4}$)-(alcoxy en $C_{2-4}$)-alkyle en $C_{2-4}$, phényle ou benzyle, et

$R_3$ représente un atome d'hydrogène ou un groupe alkyle en $C_{1-4}$ ou alkyle en $C_{1-4}$ substitué par un groupe

alcoxy en $C_{1-4}$ ou (alcoxy en $C_{1-4}$)-carbonyle.

3. Dérivés de stilbène conformes à la revendication 2, dans lesquels:

X représente un atome de chlore,

$R_1$ représente un atome d'hydrogène ou un groupe alkyle en $C_{1-2}$,

$R_2$ représente un groupe alcényle en $C_{3-4}$, cyano ou -$COOR_o$ ou -$SO_2R_x$, dans lesquels $R_o$ représente un atome d'hydrogène ou un groupe alkyle en $C_{1-4}$, et $R_x$ représente un groupe alkyle en $C_{1-4}$, et

$R_3$ représente un atome d'hydrogène ou un groupe alkyle en $C_{1-4}$.

4. Dérivés de stilbène conformes à la revendication 3, dans lesquels:

$R_1$ représente un atome d'hydrogène,

$R_2$ représente un groupe cyano ou -$COOR_o$ ou -$SO_2R_x$, dans lesquels $R_o$ représente un atome d'hydrogène ou un groupe méthyle ou éthyle et $R_x$ représente un groupe méthyle ou éthyle, et

$R_3$ représente un atome d'hydrogène.

5. Dérivés de stilbène conformes à la revendication 4, dans lequel:

$R_1$ et $R_3$ représentent chacun un atome d'hydrogène, et

$R_2$ représente un groupe cyano.

6. Procédé de préparation de dérivés de stilbène de formule:

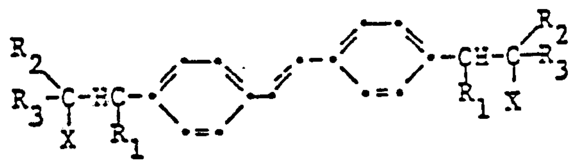

dans laquelle:

X représente un atome de chlore ou de brome,

$R_1$ représente un atome d'hydrogène ou un groupe carboxyle, cyano, alkyle en $C_{1-6}$, ou COOR, R représentant un groupe alkyle en $C_{1-4}$ ou alcényle en $C_{3-4}$, et

$R_2$ et $R_3$, indépendamment l'un de l'autre, représentent chacun un atome d'hydrogène ou un groupe alkyle en $C_{1-6}$ ou alcényle en $C_{3-4}$, non substitué ou substitué par un groupe alcoxy en $C_{1-4}$ ou (alcoxy en $C_{1-4}$)-carbonyle, ou bien un groupe cyano, carbonamido, -$COOR_o$, $R_o$ représentant un atome d'hydrogène ou un groupe alkyle en $C_{1-4}$, hydroxyalkyle en $C_{2-4}$, (alcoxy en $C_{1-4}$)-alkyle en $C_{2-4}$, (alcoxy en $C_{1-4}$)-(alcoxy en $C_{2-6}$)-alkyle en $C_{2-4}$ ou (hydroxyalcoxy en $C_{2-6}$)-alkyle en $C_{2-4}$, ou bien encore un groupe $SO_2R_x$,

$R_x$ représentant un groupe alkyle en $C_{1-4}$, (alcoxy en $C_{1-4}$)-alkyle en $C_{2-4}$, (alcoxy en $C_{1-4}$)-(alcoxy en $C_{2-4}$)-alkyle en $C_{2-4}$, phényle ou benzyle,

caractérisé en ce que l'on fait réagir 1 mole d'un composé bis-diazonium de formule:

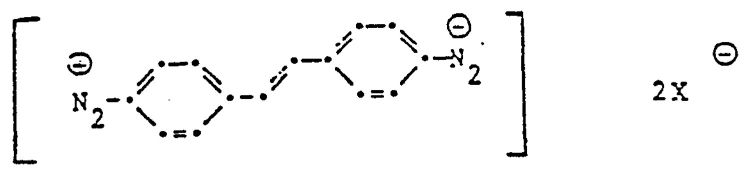

dans laquelle X représente un atome de chlore ou de brome, avec de 2 à 6 moles, et de préférence de 3 à 6 moles, d'un composé de formule:

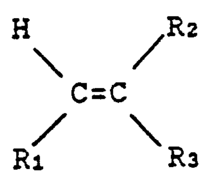

dans laquelle $R_1$, $R_2$ et $R_3$ ont les significations indiquées ci-dessus, en présence d'un halogénure de cuivre comme catalyseur et dans un solvant miscible avec l'eau.

7. Procédé conforme à la revendication 6, dans lequel:

on utilise comme catalyseur du chlorure de cuivre (I).

8. Utilisation des dérivés de stilbène obtenus conformément à la revendication 6 comme produits de départ pour la préparation d'azurants optiques de la série des stilbènes 4,4'-disubstitués.